# EUROPEAN PATENT SPECIFICATION

(11) **EP 4 321 195 B1**
(45) Date of publication and mention of the grant of the patent: **13.08.2025**
(21) Application number: 23765131.0
(22) Date of filing: 01.06.2023
(51) Int. Cl.: A61M 15/00, A61M 15/06, A61M 11/02, A61M 16/20

(54) **INHALER**
INHALATOR
INHALATEUR

(30) Priority: 01.07.2022 KR 20220081066
(43) Date of publication of application: 14.02.2024
(73) Proprietor: KT&G Corporation, Daedeok-gu Daejeon 34337 (KR)
(72) Inventor: KIM, Tae Heon, Yuseong-gu, Daejeon 34128 (KR); KIM, Jae Hyun, Yuseong-gu, Daejeon 34128 (KR); LEE, Mi Jeong, Yuseong-gu, Daejeon 34128 (KR); JEONG, Minseok, Yuseong-gu, Daejeon 34128 (KR); JUNG, Yongmi, Yuseong-gu, Daejeon 34128 (KR); JEOUNG, Eunmi, Yuseong-gu, Daejeon 34128 (KR); CHUNG, Tae Young, Yuseong-gu, Daejeon 34128 (KR); HAN, Seung Kyu, Yuseong-gu, Daejeon 34128 (KR)
(74) Representative: Ter Meer Steinmeister & Partner
(86) International application number: PCT/KR2023/007487
(87) International publication number: WO 2024/005386

(56) References cited:
- WO-A1-99/49916
- JP-A- 2014 532 445
- JP-A- 2020 151 022
- JP-B2- 2 845 887
- KR-A- 930 023 043
- US-A1- 2002 056 449
- US-A1- 2007 074 718

## Description

### Technical Field

Disclosed is an inhaler.

### Background Art

In general, an inhaler is a device used to inhale a composition such as medication through the oral cavity or nasal cavity as a liquid or gas in the process of inhalation. Such an inhaler may include a container accommodating an inhalable composition, and the composition may be sprayed from the container through a thin tube to the oral cavity or nasal cavity through an intake to be inhaled by a user.

The above description has been possessed or acquired by the inventor(s) in the course of conceiving the present disclosure and is not necessarily an art publicly known before the present application is filed. US 2007/074718 A1 relates to a medication inhaler provided for delivery of a medication to a patient from a canister having a valve stem, such as a MDI canister. The medication inhaler includes a chamber body having a fresh air inlet and a hollow spacer chamber. The inhaler further includes a canister retainer configured to move the canister between operational and stored positions, and an actuation lever for actuation of the valve stem to deliver a medication fluid into the hollow spacer chamber. In one example, the hollow spacer chamber tapers. In addition or alternatively, the chamber body includes a nozzle having an outlet, and a fresh air inlet having an outlet proximate the outlet of the nozzle. In addition or alternatively, the medication inhaler includes a canister adapter for accommodation of various size canisters. In addition or alternatively, the chamber body includes an inlet portion having a geometry for guiding the valve stem.

Prior Document: Korean Patent Gazette No. 10-2021229 (Published on September 11, 2019)

### Disclosure of the Invention

### Technical Goals

An object according to an embodiment is to provide an inhaler having a structure in which a double locking device is applied to solve a problem that a canister cover is easily opened so that a canister mounted on the inside is discharged to the outside, and a relief valve is operated to discharge a medium residue in a reservoir when the canister is replaced.

The technical tasks obtainable from the present disclosure are non-limited by the above-mentioned technical tasks. And, other unmentioned technical tasks can be clearly understood from the following description by those having ordinary skill in the technical field to which the present disclosure pertains.

### Technical Solutions

An inhaler according to the invention for achieving the above objects includes: a housing having one surface, the other surface opposite to the one surface, and a plurality of side surfaces connecting the one surface and the other surface; a canister which is mounted on inside of the housing to be replaceable and accommodates an inhalable composition; a reservoir which is connected to the canister to store the inhalable composition from the canister; a relief valve which is provided on the reservoir and operates so that the reservoir is opened or closed to the outside; and a cover having one side rotatably coupled to the housing and the other side detachably coupled to a position corresponding to a bottom surface of the canister. The cover is opened so that the canister is removed from the housing after the relief valve is opened.

According to the invention, the inhaler further includes a cover lock which is disposed adjacent to the one side of the cover, wherein the cover lock is slidable to a first position restricting rotation of the cover and a second position allowing the rotation of the cover.

According to an aspect, the inhaler may further include a relief bar having one end coupled to the relief valve and the other end extending toward the cover lock. The relief bar may be positioned between the cover and the cover lock.

According to an aspect, the cover lock may include: a first locking member which is slidable on the other surface of the housing; and a second locking member which extends from the first locking member toward the one surface of the housing. The relief bar may move toward the one surface of the housing when the first locking member moves to the second position.

According to an aspect, the second locking member may include an inclined surface having a height decreasing toward the other end of the relief bar, and when the first locking member moves to the second position, the inclined surface may move the other end of the relief bar toward the one surface of the housing.

The relief bar may open the relief valve when the relief bar moves toward the one surface of the housing, and close the relief valve when the relief bar returns to an original position.

### Effects

According to the inhaler according to an embodiment, there is an effect that a double locking device is applied to solve a problem that a canister cover is easily opened so that a canister mounted on the inside is discharged to the outside, and a relief valve is operated to discharge a medium residue in a reservoir when the canister is replaced.

The effects of the inhaler are not limited to the above-mentioned effects, and other unmentioned effects can be clearly understood from the following description by one of ordinary skill in the art to which the present disclosure pertains.

### Brief Description of Drawings

FIG. 1 is a perspective view of an inhaler according to an embodiment.
FIG. 2 is a cross-sectional view of an inhaler according to an embodiment.
FIG. 3 illustrates a cover and a cover lock of an inhaler according to an embodiment.
FIG. 4 illustrates an inhaler according to an embodiment in which a cover lock is in a first position.
FIG. 5 illustrates an inhaler according to an embodiment in which a cover lock is in a second position.

The accompanying drawings illustrate desired embodiments of the present disclosure and are provided together with the detailed description for better understanding of the technical idea of the present disclosure.

### Best Mode for Carrying Out the Invention

Hereinafter, embodiments will be described in detail with reference to the illustrative drawings. Regarding the reference numerals assigned to the components in the drawings, it should be noted that the same components will be designated by the same reference numerals, wherever possible, even though they are shown in different drawings. Further, in the following description of the present embodiments, a detailed description of publicly known configurations or functions incorporated herein will be omitted when it is determined that the detailed description obscures the subject matters of the present embodiments.

In addition, the terms first, second, A, B, (a), and (b) may be used to describe constituent elements of the embodiments. These terms are used only for the purpose of discriminating one constituent element from another constituent element, and the nature, the sequences, or the orders of the constituent elements are not limited by the terms. When one constituent element is described as being "connected", "coupled", or "attached" to another constituent element, it should be understood that one constituent element can be connected or attached directly to another constituent element, and an intervening constituent element can also be "connected", "coupled", or "attached" to the constituent elements.

The same name may be used to describe an element included in the embodiments described above and an element having a common function. Unless otherwise mentioned, the descriptions on the embodiments may be applicable to the following embodiments and thus, duplicated descriptions will be omitted for conciseness.

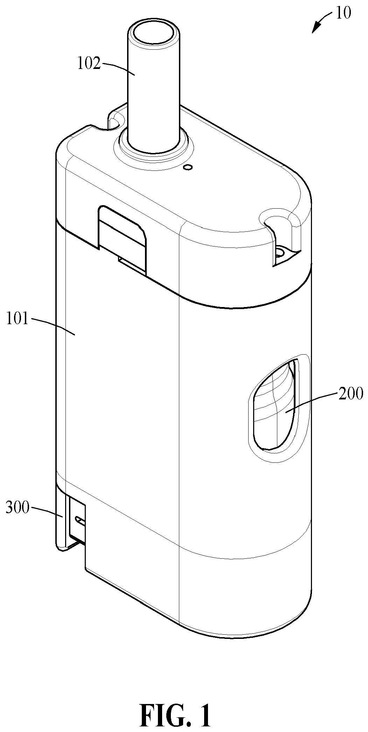

FIG. 1 is a perspective view of an inhaler 10 according to an embodiment.

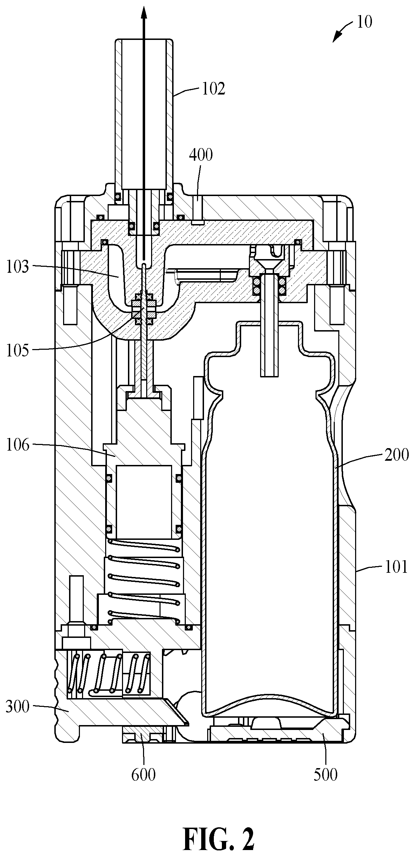

FIG. 2 is a cross-sectional view of the inhaler 10 according to an embodiment.

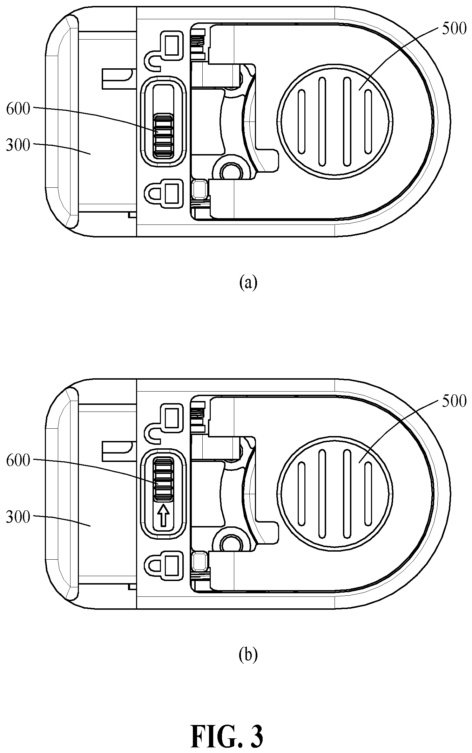

FIG. 3 illustrates a cover 500 and a cover lock 600 of the inhaler 10 according to an embodiment.

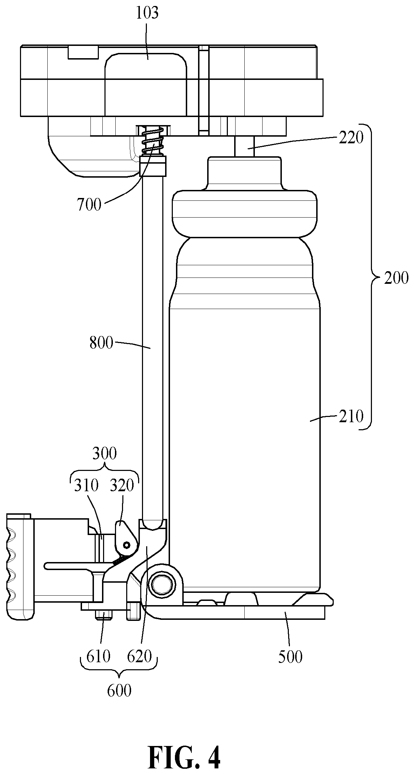

FIG. 4 illustrates the inhaler 10 according to an embodiment in which the cover lock 600 is in a first position.

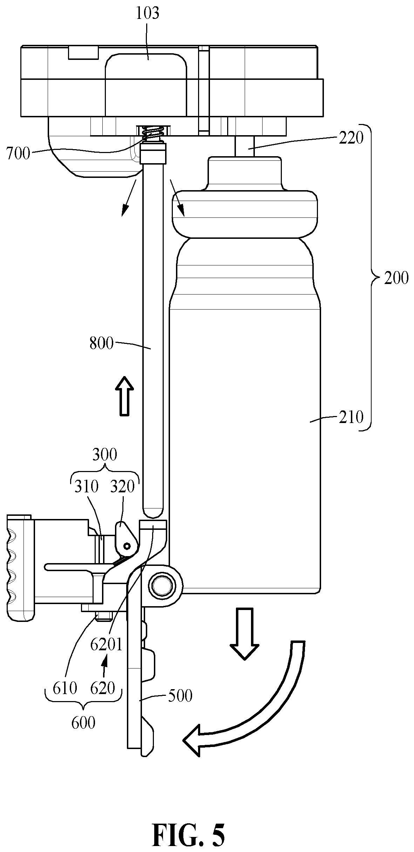

FIG. 5 illustrates the inhaler 10 according to an embodiment in which the cover lock 600 is in a second position.

Referring to FIG. 1, the inhaler 10 according to an embodiment may include a housing 101, a mouthpiece 102, a reservoir 103, a canister 200, and a lever 300.

The housing 101 may include a first surface formed on one surface, a second surface opposite to the first surface, and a plurality of side surfaces connecting the first surface and the second surface. The first surface of the housing 101 may be, for example, a surface located on the top of the housing 101, and the second surface may be, for example, a bottom surface of the housing 101.

The mouthpiece 102 may be disposed on the first surface of the housing 101. A user may inhale an inhalable composition accommodated in the inhaler 10 through the mouthpiece 102. At this time, the user may inhale the composition, for example, in the form of an aerosol or in the form of a powder. Hereinafter, the inhaler 10 according to an embodiment will be described using the inhaler 10 that sprays an inhalable composition in the form of an aerosol as an example.

Referring to FIGS. 1 and 2, a reservoir 103 may be disposed inside the housing 101. Since one side of the canister 200 is connected to the reservoir 103, the inhalable composition accommodated in the canister 200 may be moved to and stored in the reservoir 103. In addition, as the user applies a suction force through the mouthpiece 102, the inhalable composition stored in the reservoir 103 may be discharged through the mouthpiece 102 in the form of an aerosol, and accordingly, the inhalable composition stored in the reservoir 103 may be gradually consumed.

The canister 200 may be mounted inside the housing 101. In this case, the canister 200 may be mounted interchangeably inside the housing 101. In addition, the canister 200 may accommodate an inhalable composition therein. A certain amount of the composition of the canister 200 may be filled in the reservoir 103.

The inhaler 10 according to an embodiment may further include the cover 500 and the cover lock 600 for preventing the canister 200 mounted on the housing 101 from being separated.

The cover 500 and the cover lock 600 will be described in more detail with reference to FIGS. 3 to 5 below.

Referring back to FIGS. 1 and 2, the lever 300 may be pressed by the user to fill the inside of the reservoir 103 with the composition accommodated in the canister 200. The lever 300 may form one side surface of the housing 101 and may be positioned adjacent to the second surface. When the user presses the lever 300, the lever 300 may push up a bottom surface of the canister 200 so that an injection hole 220 of the canister 200 communicates with the reservoir 103, and the composition may move from the canister 200 to the reservoir 103 through the injection hole 220.

Hereinafter, a direction from one side surface of the housing 101 where the lever 300 is located to the canister 200 is defined as a first direction, and a direction from the second surface of the housing 101 to the first surface is defined as a second direction.

Meanwhile, the relief valve 700 may be provided on the reservoir 103, and a relief vent hole 400 may be provided on the first surface of the housing 101. The relief valve 700 may be interlocked with the lever 300 through a relief bar 800, a relief bar movement protrusion 320, or the like, and the relief valve 700 may operate to open before the lever 300 pushes the canister 200 up. Accordingly, a residual gas in the reservoir 103 may be discharged before the reservoir 103 is filled with the inhalable composition.

In addition, in the inhaler 10 according to an embodiment, the user may visually identify a remaining amount of the composition stored in the reservoir 103 through a viewing window (not shown) provided on the housing 101. In addition, the inhaler 10 according to an embodiment may include the counter (not shown) that counts the number of times of filling in connection with a vertical movement of the canister 200 during the filling of the reservoir 103, and a display window (not shown) that displays a remaining amount of the composition in the canister 200. The composition stored in the reservoir 103 may be sprayed in the form of an aerosol as the user applies a suction force to the mouthpiece 102. At this time, an inhalation interlocking valve 105 that opens and closes the reservoir 103 by the suction force may operate, and the opening and closing operation of the inhalation interlocking valve 105 may be controlled by a piston 106.

As described above, the canister 200 may be mounted on the inhaler 10 according to an embodiment to be replaceable. Accordingly, when the inhalable composition in the canister 200 is used up, the user may replace the existing canister 200 with a new canister 200. For this, the cover 500 of the canister 200 is opened, however, when the cover 500 is opened in a state where the inhalable composition remains in the reservoir 103, the composition may be sprayed from the injection hole 220 of the canister 200 and push the canister 200 with a strong force. At this time, a problem that the canister 200 is discharged and a problem that the composition wets the user's hand may occur. To solve such problems, the cover lock 600 as a double locking device may be applied to the inhaler 10 according to an embodiment.

Referring to FIG. 3, the cover lock 600 of the inhaler 10 according to an embodiment may be positioned on the second surface of the housing 101. The cover lock 600 may be provided to be slidable, and may be moved to a position shown in FIG. 3A or 3B by the user.

For example, FIG. 3A shows the cover lock 600 in the first position, and in the first position, the cover lock 600 may restrict the rotation of the cover 500 so that the cover 500 is not opened. FIG. 3B shows the cover lock 600 moved to the second position, and in the second position, the cover lock 600 may allow the rotation of the cover 500 so that the cover 500 is opened. When the cover 500 is opened, the user may remove the canister 200 from the housing 101 of the inhaler 10.

Referring to FIGS. 4 and 5, the cover 500 may have one side rotatably coupled to the housing 101, and the other side detachably coupled to the housing 101 at a position corresponding to the bottom surface of the canister 200. The other side of the cover 500 may be separated from the housing 101 and the one side thereof may rotate to expose the bottom surface of the canister 200 to the outside. The rotation of the one side of the cover 500 may be controlled by the cover lock 600 as described above.

In addition, the cover lock 600 may first open the relief valve 700 before allowing the cover 500 to rotate.

Referring back to FIGS. 4 and 5, the relief valve 700 of the inhaler 10 according to an embodiment may be provided on the reservoir 103 to be spaced apart from the one side of the canister 200. This relief valve 700 may be opened or closed to allow the reservoir 103 to communicate with the outside or block the reservoir 103 from the outside.

For example, the relief valve 700 may be opened by the relief bar 800 that is raised when the cover lock 600 is in the second position.

Specifically, the inhaler 10 according to an embodiment may further include the relief bar 800 having one end coupled to the relief valve 700 and the other end extending toward the cover lock 600. The relief bar 800 may be positioned between the cover 500 and the cover lock 600.

The relief bar 800 may open the relief valve 700 as the relief bar 800 moves in the second direction, and may close the relief valve 700 as the relief bar 800 returns to its original position.

The movement of the relief bar 800 may be controlled by the cover lock 600.

As shown in FIG. 4, when the cover lock 600 is in the first position, the relief bar 800 does not rise and the relief valve 700 may remain closed. On the other hand, as shown in FIG. 5, when the cover lock 600 is in the second position, the relief bar 800 may be raised by the cover lock 600, and the relief valve 700 may be opened.

The cover lock 600 of the inhaler 10 according to an embodiment may include a first locking member 610 and a second locking member 620.

The first locking member 610 may slide on the second surface of the housing 101.

The second locking member 620 may extend from the first locking member 610 toward the first surface of the housing 101. The second locking member 620 may push up the other end of the relief bar 800 toward the first surface of the housing 101 when the first locking member 610 moves from the first position to the second position.

Specifically, one end of the second locking member 620 may be fixed to the first locking member 610, and the other end of the second locking member 620 may extend toward the first surface of the housing 101. An inclined surface 6201 having a height decreasing toward the relief bar 800 may be formed on the other end of the second locking member 620.

When the first locking member 610 moves from the first position to the second position, the inclined surface 6201 may gradually move the other end of the relief bar 800 toward the first surface of the housing 101. When the relief bar 800 rises toward the first surface of the housing 101 as described above, the relief valve 700 may be opened. In addition, when the first locking member 610 moves from the second position to the first position, the second locking member 620 including the inclined surface 6201 may return to its original position and the relief bar 800 may move down toward the second surface of the housing 101, thereby closing the relief valve 700.

As a result, when the first locking member 610 moves from the first position to the second position, the relief valve 700 may be opened, the remaining composition in the reservoir 103 may be discharged, and the canister 200 may also be removed from the housing 101. Also, when the first locking member 610 is moved to the first position again, the relief valve 700 may be closed.

Meanwhile, when the first locking member 610 is in the first position, the second locking member 620 does not restrict the operation of the lever 300, but when the first locking member 610 is in the second position, the second locking member 620 may restrict the operation of the lever 300.

The lever 300 may include a support member 310 and a protrusion member 320.

The support member 310 may extend toward the canister 200.

The protrusion member 320 may be rotatably coupled to the support member 310 around a rotation shaft provided in the support member 310. The protrusion member 320 may have an end portion protruding in a radial direction from the rotation shaft at one side. The end portion of the protrusion member 320 may be disposed in a standing state toward the first surface of the housing 101.

The other end of the relief bar 800 may be positioned between the protrusion member 320 and the second surface of the housing 101. In addition, the end portion of the protrusion member 320 may be inclined in a direction away from the canister 200 with respect to the rotation shaft.

When the lever 300 is pressed in the first direction, the support member 310 may restrain the rotation of the protrusion member 320. That is, the rotation of the protrusion member 320 in a direction away from the canister 200 may be restrained in a state where the end portion stands toward the first surface of the housing 101.

Therefore, when the lever 300 is pressed, the protrusion member 320 also moves in the first direction to reach the first position, and at this time, the protrusion member 320 may come into contact with the relief bar 800 and push the relief bar 800 up in the second direction without rotating.

As described above, the opening or closing operation of the relief valve 700 may be controlled by the lever 300. For example, when the lever 300 is pressed by the user, the lever 300 may control the relief valve 700 so that the relief valve 700 is opened first and then the canister 200 is opened.

As described above, as the relief valve 700 interlocked with the lever 300 is applied to the inhaler 10 according to an embodiment, the residual gas in the reservoir 103 may be discharged and the reservoir 103 may be smoothly filled with the inhalable composition from the canister 200.

However, the operation of the lever 300 with respect to the relief valve 700 may be allowed when the first locking member 610 of the cover lock 600 is in the first position, as described above.

Referring to FIG. 5, when the first locking member 610 of the cover lock 600 is in the second position, the second locking member 620 may block a path of the lever 300 moving in the first direction. For example, when the first locking member 610 is in the second position, the second locking member 620 may be moved adjacent to the protrusion member 320, thereby blocking the moving path of the protrusion member 320. At the same time, the second locking member 620 may raise the relief bar 800. Accordingly, although the operation in which the lever 300 raises the relief bar 800 to open the relief valve 700, and then raises the canister 200 to open the injection hole 220 is not performed, the cover lock 600 may open only the relief valve 700 to discharge the remaining composition in the reservoir 103.

As described above, in the inhaler 10 according to an embodiment, the cover lock 600 may be applied as a double locking device to solve a problem that the canister cover 500 is easily opened so that the canister 200 mounted on the inside is discharged to the outside or the composition wets the user's hands. In addition, when the canister 200 is replaced, the inhaler 10 according to an embodiment may operate the relief valve 700 to discharge a medium residue in the reservoir 103.

In addition, in the inhaler 10 according to an embodiment, when the cover lock 600 is in the first position, the cover 500 does not open and the filling lever 300 may normally operate, and when the cover lock 600 is in the second position, only the cover 500 may be opened and the filling lever 300 may be caught by the second locking member 620 and may not operate. In addition, when the first locking member 610 is moved to the second position, the inclined surface 6201 extending from the second locking member 620 may push the relief bar 800 up to open the relief valve 700 and discharge the residue in the reservoir.

While the embodiments of the present disclosure have been described above with reference to specific components, and limited embodiments and drawings, the above descriptions are merely for better understanding of the present disclosure. Accordingly, the scope of the present disclosure is defined not by the detailed description, but by the appended claims, and all variations within the scope of the claims are to be construed as being included in the disclosure.

## Claims

1. An inhaler (10) comprising:
a housing (101) having one surface, the other surface opposite to the one surface, and a plurality of side surfaces connecting the one surface and the other surface;
a canister (200) which is mounted on inside of the housing (101) to be replaceable and accommodates an inhalable composition;
a reservoir (103) which is connected to the canister (200) to store the inhalable composition from the canister (200);
a relief valve (700) which is provided on the reservoir (103) and operates so that the reservoir (103) is opened or closed to the outside;
a cover (500) having one side rotatably coupled to the housing (101) and the other side detachably coupled to a position corresponding to a bottom surface of the canister (200); and
a cover lock (600) which is disposed adjacent to the one side of the cover (500),
wherein the cover (500) is opened so that the canister (200) is removed from the housing (101) after the relief valve (700) is opened, and
wherein the cover lock (600) is slidable to a first position restricting rotation of the cover (500) and a second position allowing the rotation of the cover (500).

2. The inhaler (10) of claim 1, further comprising:
a relief bar (800) having one end coupled to the relief valve (700) and the other end extending toward the cover lock (600),
wherein the relief bar (800) is positioned between the cover (500) and the cover lock (600).

3. The inhaler (10) of claim 2,
wherein the cover lock (600) comprises:
a first locking member (610) which is slidable on the other surface of the housing (101); and
a second locking member (620) which extends from the first locking member (610) toward the one surface of the housing (101), and
wherein the relief bar (800) moves toward the one surface of the housing (101) when the first locking member (610) moves to the second position.

4. The inhaler (10) of claim 3,
wherein the second locking member (620) comprises an inclined surface (6201) having a height decreasing toward the other end of the relief bar (800), and
wherein, when the first locking member (610) moves to the second position, the inclined surface (6201) moves the other end of the relief bar (800) toward the one surface of the housing (101).

5. The inhaler (10) of claim 2, wherein the relief bar (800) opens the relief valve (700) when the relief bar (800) moves toward the one surface of the housing (101), and closes the relief valve (700) when the relief bar (800) returns to an original position.

## Patentansprüche

1. Inhalator (10), der umfasst:
ein Gehäuse (101) mit einer Fläche, einer anderen Fläche gegenüber der einen Fläche und mehreren Seitenflächen, die die eine Fläche und die andere Fläche verbinden;
einen Behälter (200), der so innerhalb des Gehäuses (101) befestigt ist, dass er austauschbar ist, und der eine inhalierbare Zusammensetzung aufnimmt;
ein Reservoir (103), das mit dem Behälter (200) verbunden ist, um die inhalierbare Zusammensetzung von dem Behälter (200) zu lagern;
ein Entlastungsventil (700), das an dem Reservoir (103) vorgesehen ist und so arbeitet, dass das Reservoir (103) nach außen geöffnet oder geschlossen wird;
eine Abdeckung (500) mit einer Seite, die drehbar an das Gehäuse (101) gekoppelt ist, und der anderen Seite, die abnehmbar an einer Position gekoppelt ist, die einer unteren Fläche des Behälters (200) entspricht; und
einen Abdeckungsverschluss (600), der angrenzend an die eine Seite der Abdeckung (500) angeordnet ist,
wobei die Abdeckung (500) geöffnet wird, so dass der Behälter (200) von dem Gehäuse (101) entfernt wird, nachdem das Entlastungsventil (700) geöffnet worden ist, und
wobei der Abdeckungsverschluss (600) in eine erste Position, die eine Drehung der Abdeckung (500) begrenzt, und in eine zweite Position, die die Drehung der Abdeckung (500) erlaubt, gleiten kann.

2. Inhalator (10) nach Anspruch 1, der ferner umfasst:
eine Entlastungsschiene (800) mit einem Ende, das an das Entlastungsventil (700) gekoppelt ist, und dem anderen Ende, das sich in Richtung des Abdeckungsverschlusses (600) erstreckt,
wobei die Entlastungsschiene (800) zwischen der Abdeckung (500) und dem Abdeckungsverschluss (600) positioniert ist.

3. Inhalator (10) nach Anspruch 2,
wobei der Abdeckungsverschluss (600) umfasst:
ein erstes Verriegelungselement (610), das auf der anderen Fläche des Gehäuses (101) gleiten kann; und
ein zweites Verriegelungselement (620), das sich von dem ersten Verriegelungselement (610) in Richtung der einen Fläche des Gehäuses (101) erstreckt,
wobei sich die Entlastungsschiene (800) in Richtung der einen Fläche des Gehäuses (101) bewegt, wenn sich das erste Verriegelungselement (610) in die zweite Position bewegt.

4. Inhalator (10) nach Anspruch 3,
wobei das zweite Verriegelungselement (620) eine geneigte Fläche (6201) mit einer Höhe, die in Richtung des anderen Endes der Entlastungsschiene (800) abnimmt, umfasst und
wobei dann, wenn sich das erste Verriegelungselement (610) in die zweite Position bewegt, die geneigte Fläche (6201) das andere Ende der Entlastungsschiene (800) in Richtung der einen Fläche des Gehäuses (101) bewegt.

5. Inhalator (10) nach Anspruch 2, wobei die Entlastungsschiene (800) das Entlastungsventil (700) öffnet, wenn sich die Entlastungsschiene (800) in Richtung der einen Fläche des Gehäuses (101) bewegt, und das Entlastungsventil (700) schließt, wenn die Entlastungsschiene (800) in ihre Ausgangsposition zurückkehrt.

## Revendications

1. Inhalateur (10) comportant :
un boîtier (101) ayant une première surface, la seconde surface étant opposée à la première surface, et une pluralité de surfaces latérales reliant la première surface et la seconde surface ;
une cartouche (200) qui est montée sur un intérieur du boîtier (101) de manière à pouvoir être remplacée et qui reçoit une composition inhalable ;
un réservoir (103) qui est relié à la cartouche (200) pour stocker la composition inhalable provenant de la cartouche (200) ;
une soupape de décharge (700) qui est disposée sur le réservoir (103) et qui fonctionne de sorte que le réservoir (103) est ouvert ou fermé par rapport à l'extérieur ;
un couvercle (500) ayant un premier côté couplé en rotation au boîtier (101) et le second côté couplé de manière détachable à une position correspondant à une surface inférieure de la cartouche (200) ; et
un verrou de couvercle (600) qui est disposé au voisinage du premier côté du couvercle (500),
dans lequel le couvercle (500) est ouvert de sorte que la cartouche (200) est retirée du boîtier (101) après l'ouverture de la soupape de décharge (700), et
dans lequel le verrou de couvercle (600) peut coulisser jusqu'à une première position restreignant une rotation du couvercle (500) et une seconde position permettant la rotation du couvercle (500).

2. Inhalateur (10) selon la revendication 1, comportant en outre :
une barrette de décharge (800) ayant une extrémité couplée à la soupape de décharge (700) et l'autre extrémité s'étendant vers le verrou de couvercle (600),
dans lequel la barrette de décharge (800) est positionnée entre le couvercle (500) et le verrou de couvercle (600).

3. Inhalateur (10) selon la revendication 2,
dans lequel le verrou de couvercle (600) comporte :
un premier élément de verrouillage (610) qui peut coulisser sur la seconde surface du boîtier (101) ; et
un second élément de verrouillage (620) qui s'étend à partir du premier élément de verrouillage (610) vers la première surface du boîtier (101), et
dans lequel la barrette de décharge (800) se déplace vers la première surface du boîtier (101) lorsque le premier élément de verrouillage (610) se déplace jusqu'à la seconde position.

4. Inhalateur (10) selon la revendication 3,
dans lequel le second élément de verrouillage (620) comporte une surface inclinée (6201) ayant une hauteur diminuant vers l'autre extrémité de la barrette de décharge (800), et
dans lequel, lorsque le premier élément de verrouillage (610) se déplace jusqu'à la seconde position, la surface inclinée (6201) déplace l'autre extrémité de la barrette de décharge (800) vers la première surface du boîtier (101).

5. Inhalateur (10) selon la revendication 2, dans lequel la barrette de décharge (800) ouvre la soupape de décharge (700) lorsque la barrette de décharge (800) se déplace vers la première surface du boîtier (101), et ferme la soupape de décharge (700) lorsque la barrette de décharge (800) revient à une position d'origine.
